# EUROPÄISCHE PATENTSCHRIFT

(11) **EP 0 673 940 B1**
(45) Veröffentlichungstag und Bekanntmachung des Hinweises auf die Patenterteilung: **02.05.2002**
(21) Anmeldenummer: 95810175.0
(22) Anmeldetag: 16.03.1995
(51) Int. Cl.: C07D 487/04, C08K 5/3415, C09B 57/00

(54) **Cyaniminogruppen enthaltende Pyrrolo(3,4-c)pyrrole**
Pyrrolo (3,4-c) pyrroles substituted by cyaniminogroups
Pyrrolo (3,4-c) pyrroles substitués par groupe cyanimino

(30) Priorität: 25.03.1994 CH 91594
(43) Veröffentlichungstag der Anmeldung: 27.09.1995
(73) Patentinhaber: Ciba Specialty Chemicals Holding Inc., 4057 Basel (CH)
(72) Erfinder: Zambounis, John S., Dr., CH-3280 Murten (CH); Hao, Zhimin, Dr., CH-1723 Marly (CH); Iqbal, Abul, Dr., CH-1732 Arconciel (CH)

(56) Entgegenhaltungen:
- EP-A- 0 133 156

## Beschreibung

Die vorliegende Erfindung betrifft neue 1-Keto-4-cyanimino-pyrrolo[3,4-c]pyrrole und 1,4-Dicyanimino-pyrrolo[3,4-c]pyrrole, ihre Herstellung und ihre Verwendung zum Färben von hochmolekularem organischem Material.

1,4-Diketopyrrolo[3,4-c]pyrrole sind seit einigen Jahren bekannt und werden z.B. in US-Patent 4 415 685 und US-Patent 4 579 949 als wertvolle Pigmente beschrieben. Einige davon haben sich bereits in der Praxis als hochwertige Pigmente bewährt. Aus US-Patent 4 585 878 sind N-substituierte 1,4-Diketopyrrolo[3,4-c]pyrrole bekannt, welche je nach Art ihrer Substituenten als polymerlösliche Farbstoffe oder als Pigmente eingesetzt werden können. Wenn diese in den angewandten Polymeren gelöst vorliegen, zeichnen sie sich u.a. auch durch hohe Fluoreszenz aus. Dieses gleiche Verhalten zeigen auch die allerdings N-unsubstituierten 1-Keto-4-imino-pyrrolo[3,4-c]pyrrole, die in US-Patent 5 017 706 beschrieben sind.

Gemäss der vorliegenden Erfindung sind nun neuartige 1-Keto-4-cyanimino-pyrrolo-[3,4-c]pyrrole bzw. 1,4-Dicyanimino-pyrrolo[3,4-c]pyrrole mit überraschend guten Eigenschaften gefunden worden, dank denen sie ganz ausgezeichnet als Farbmittel, d.h. als Pigmente oder als polymerlösliche Farbstoffe, zum Einfärben von hochmolekularem organischen Material verwendet werden können. Charakteristisch für diese neuen, durch den Ersatz der Keto- durch Cyaniminogruppe(n) erhaltenen Verbindungen ist u.a. eine bathochrome Nuancenverschiebung und, ganz überraschend, eine hohe Festkörperfluoreszenz.

Die vorliegende Erfindung betrifft demnach Pyrrolo[3,4-c]pyrrole der Formel 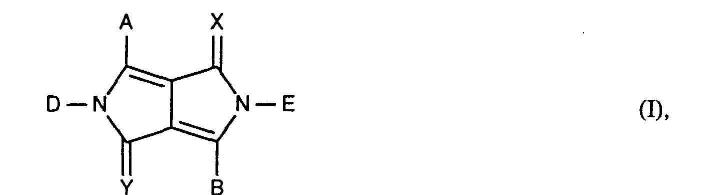 worin A und B unabhängig voneinander für eine Gruppe der Formel 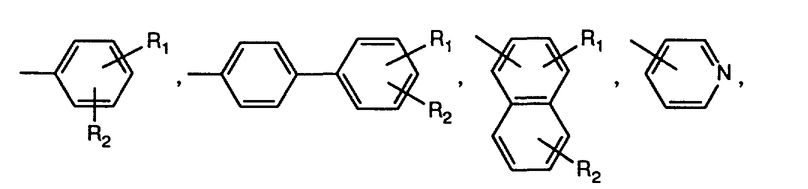 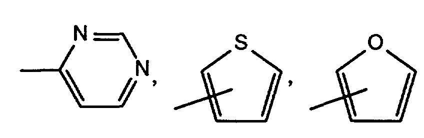 oder 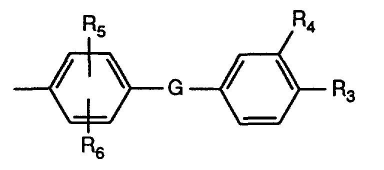 stehen, worin
R₁ und R₂ unabhängig voneinander Wasserstoff, Halogen, C₁-C₁₈-Alkyl, C₁-C₁₈-Alkoxy, C₁-C₁₈-Alkylmercapto, C₁-C₁₈-Alkylamino, -CN, -NO₂, -Phenyl, Trifluormethyl, C₅-C₆-Cycloalkyl, -C=N-(C₁-C₁₈-Alkyl), 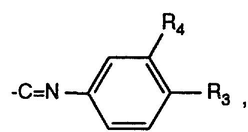 Imidazolyl, Pyrazolyl, Triazolyl, Piperazinyl, Pyrrolyl, Oxazolyl, Benzoxazolyl, Benzthiazolyl, Benzimidazolyl, Morpholinyl, Piperidinyl oder Pyrrolidinyl,
R₃ und R₄ unabhängig voneinander Wasserstoff, Halogen, C₁-C₆-Alkyl, C₁-C₁₈-Alkoxy oder -CN,
R₅ und R₆ unabhängig voneinander Wasserstoff, Halogen oder C₁-C₆-Alkyl, und
G -CH₂-, -CH(CH₃)-, -(CH₃)₂-, -CH=N-, -N=N-, -O-, -S-, -SO-, -SO₂- oder -NR₇-, worin R₇ Wasserstoff oder C₁-C₆-Alkyl ist, bedeuten,
D und E unabhängig voneinander Wasserstoff, C₁-C₁₈-Alkyl, C₂-C₄-Alkenyl, C₇-C₁₀-Aralkyl, unsubstituiertes oder durch Chlor, Brom, C₁-C₆-Alkyl, C₁-C₄-Alkoxy, Trifluormethyl oder Nitro substituiertes Phenyl, -COO-C₁-C₅-Alkyl oder eine Gruppe -COOR₈,
worin R₈ Benzyl, Piperidyl oder eine Gruppe 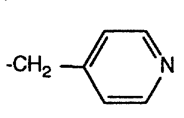 oder 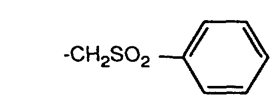 ist, bedeuten, und
X und Y für N-CN oder O stehen, wobei mindestens einer von X oder Y N-CN sein muss.

Bedeuten etwaige Substituenten Halogen, dann handelt es sich z.B. um Jod, Fluor, insbesondere Brom und bevorzugt Chlor;
bei C₁-C₆-Alkyl handelt es sich beispielsweise um Methyl, Ethyl, n-Propyl, Isopropyl, n-Butyl, sec.-Butyl, tert.-Butyl, n-Amyl, tert.-Amyl, Hexyl und bei C₁-C₁₈-Alkyl zusätzlich z.B. um Heptyl, Octyl, 2-Ethylhexyl, Nonyl, Decyl, Dodecyl, Tetradecyl, Hexadecyl oder Octadecyl;
C₁-C₄-Alkoxy bedeutet z.B. Methoxy, Ethoxy, n-Propoxy, Isopropoxy, Butyloxy, und C₁-C₁₈-Alkoxy zusätzlich z.B. Hexyloxy, Decyloxy, Dodecyloxy, Hexadecyloxy oder Octadecyloxy;
C₁-C₁₈-Alkylmercapto steht beispielsweise für Methylmercapto, Ethylmercapto, Propylmercapto, Butylmercapto, Octylmercapto, Decylmercapto, Hexadecylmercapto oder Octadecylmercapto;
C₁-C₁₈-Alkylamino bedeutet z.B. Methylamino, Ethylamino, Propylamino, Hexylamino, Decylamino, Hexadecylamino oder Octadecylamino.

C₃-C₆-Cycloalkyl steht z.B. für Cyclopropyl, Cyclopentyl und insbesondere für Cyclohexyl.

C₂-C₄-Alkenyl bedeutet z.B. Vinyl, Allyl, Methallyl, 2-Butenyl.

C₇-C₁₀-Aralkyl steht z.B. für 1-Phenethyl, 1,1-Dimethylbenzyl, im Benzolkern durch Methyl oder Ethyl substituiertes Benzyl oder vor allem für Benzyl.

COO-C₁-C₅-Alkyl bedeutet beispielsweise Methoxy-, Ethoxy-, Isopropoxy-, n-Butoxy-, tert.-Amyloxy- oder insbesondere tert.-Butoxycarbonyl.

Von besonderem Interesse sind Pyrrolo[3,4-c]pyrrole der Formel I, worin A und B unabhängig voneinander eine Gruppe der Formel 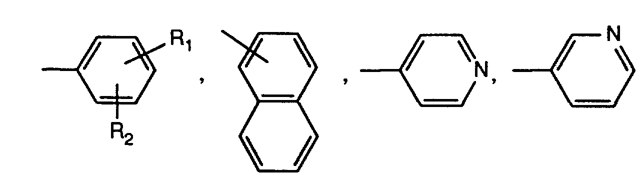 oder 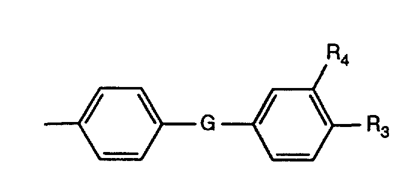 sind,
worin R₁ und R₂ unabhängig voneinander Wasserstoff, Chlor, Brom, C₁-C₄-Alkyl, C₁-C₆-Alkoxy, C₁-C₆-Alkylamino, CN oder Phenyl,
R₃ und R₄ Wasserstoff, und
G -O-, -NR₇-, -N=N- oder -SO₂-, worin R₇ Wasserstoff, Methyl oder Ethyl ist, bedeuten,
wobei vorzugsweise A und B gleich sind,
und insbesondere jene, worin A und B eine Gruppe der Formel 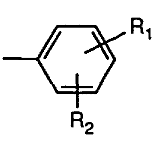 sind,
worin R₁ und R₂ unabhängig voneinander Wasserstoff, Methyl, tert.-Butyl, Chlor, Brom, CN oder Phenyl bedeuten. R₂ ist bevorzugt Wasserstoff.

Bevorzugt sind Pyrrolo[3,4-c]pyrrole der Formel I, worin D und E gleich sind und C₁-C₄-Alkyl, unsubstituiertes oder durch Chlor substituiertes Phenyl, Allyl oder Benzyl oder eine Gruppe -COO-C(CH₃)₃, 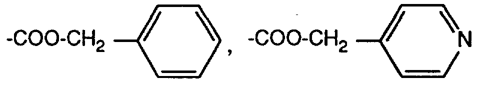 oder 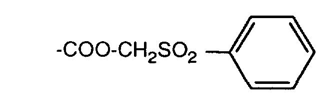 bedeuten.

D und E bedeuten vorzugsweise C₁-C₄-Alkyl oder eine Gruppe -COO-C(CH₃)₃ und X und Y die Gruppe N-CN.

Die Herstellung der erfindungsgemässen Pyrrolo[3,4-c]pyrrole der Formel I, worin A, B, X und Y die oben angegebene Bedeutung haben und D und E unabhängig voneinander Wasserstoff C₁-C₁₈-Alkyl, C₂-C₄-Alkenyl, C₇-C₁₀-Aralkyl, unsubstituiertes oder durch Chlor, Brom C₁-C₆-Alkyl, C₁-C₄-Alkoxy, Trifluormethyl oder Nitro substituiertes Phenyl bedeuten, erfolgt in Anlehnung an eine von S. Hünig et al. in Angew. Chem. 96, 437, 1984 und 102, 220, 1990, beschriebenen Methode ganz überraschend mit guter Ausbeute durch Umsetzung eines Pyrrolo[3,4-c]pyrrols der Formel 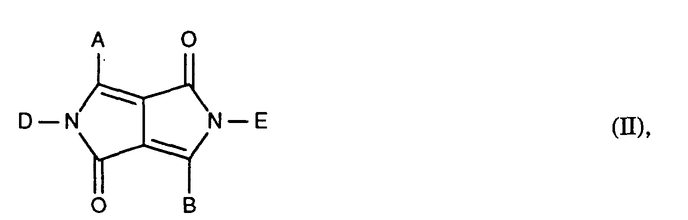 worin A und B die oben und D und E die soeben angegebene Bedeutung haben, im gewünschten Molverhältnis mit einer Verbindung der Formel

(R₉)₃SiN=C=NSi(R₉)₃ (III)

oder

NH₂CN (IV)

wobei R₉ C₁-C₆-Alkyl bedeutet,
in Gegenwart einer Lewis-Säure als Katalysator in einem aprotischen organischen Lösungsmittel, bei Temperaturen zwischen 10 und 150°C, bevorzugt zwischen 50 und 100°C.

Die Dauer der Umsetzung variiert je nach Substituenten von etwa 30 Minuten bis etwa 200 Stunden.

Die von Hünig et al. beschriebene Methode betrifft ausschliesslich die Umsetzung von löslichen Ausgangsprodukten. Dass die Reaktion auch ausgehend von unlöslichen Diketopyrrolo[3,4-c]pyrrolen so erfolgreich verlaufen könnte, war nicht zu erwarten. Dieses neue Herstellungsverfahren ist demnach auch Gegenstand der vorliegenden Erfindung.

Die Cyaniminogruppen liefernde Verbindung III oder IV wird zweckmässig stöchiometrisch bis in einem ca. 10- bis 20-fachen Überschuss, bezogen auf das Pyrrolopyrrol, eingesetzt. Der erwähnte Überschuss ist bevorzugt.

Geeignete, als Katalysator einzusetzende Lewis-Säuren sind beispielsweise CsF, BF₃, ZrCl₄ und insbesondere TiCl₄.

Als Lösungsmittel eignen sich beispielsweise Ether, wie Tetrahydrofuran oder Dioxan, oder Glykoläther, wie Ethylenglykol-methyläther, Ethylenglykol-ethylether, Diethylenglykol-monomethylether oder Diethylenglykol-monoethylether, ferner dipolar-aprotische Lösungsmittel, wie Acetonitril, Benzonitril, N,N-Dimethylformamid, N,N-Dimethylacetamid, Nitrobenzol, N-Methylpyrrolidon, halogenierte aliphatische oder aromatische Kohlenwasserstoffe, wie Trichlorethan, Benzol oder durch Alkyl, Alkoxy oder Halogen substituiertes Benzol, wie Toluol, Xylol, Anisol oder Chlorbenzol oder aromatische N-Heterocyclen, wie Pyridin, Picolin oder Chinolin. Bevorzugte Lösungsmittel sind z.B. Tetrahydrofuran, N,N-Dimethylformamid, N-Methylpyrrolidon. Die genannten Lösungsmittel können auch als Mischungen eingesetzt werden. Zweckmässigerweise verwendet man 5-20 Gew.-Teile Lösungsmittel auf 1 Gew.-Teil der Reaktionsteilnehmer.

Pyrrolo[3,4-c]pyrrole der Formel II sind bekannte Verbindungen. Sollten einige noch neu sein, so können sie nach allgemein bekannten Methoden hergestellt werden.
Pyrrolo[3,4-c]pyrrole der Formel I, worin D und/oder E -COO-C₁-C₅-Alkyl oder eine Gruppe -COOR₈ bedeuten, können in Analogie zu bekannten Methoden, z.B. durch Umsetzung eines Pyrrolo[3,4-c]pyrrols der Formel I, worin D und E Wasserstoff bedeuten, im gewünschten Molverhältnis mit einem Dicarbonat der Formel

E-O-E (V)

oder mit einem 1:1 Gemisch von Dicarbonat der Formel V und Dicarbonat der Formel

D - O - D (VI),

worin E und D unabhängig voneinander -COO-C₁-C₅-Alkyl oder eine Gruppe -COOR₈, worin R₈ die oben angegebene Bedeutung hat, bedeuten, hergestellt werden.

Bei den Verbindungen der Formeln III, IV, V und VI handelt es sich um bekannte und im Handel erhältliche Verbindungen.

Je nach Art ihrer Substituenten und des zu färbenden Polymeren können die Pyrrolo-[3,4-c]pyrrole der Formel I, wie die entsprechenden in US-Patent 4 585 878 beschriebenen 1,4-Diketopyrrolo[3,4-c]pyrrole als polymerlösliche Farbstoffe für z.B. Polystyrol, Polyamide, ABS und insbesondere lineare Polyester, oder auch als Pigmente für hochmolekulares organisches Material verwendet werden. Im Vergleich zu den 1,4-Diketopyrrolo[3,4-c]pyrrolen zeichnen sich die erfindungsgemässen Pyrrolo[3,4-c]pyrrolen insbesondere durch eine überraschend hohe Festkörperfluoreszenz sowie durch eine koloristisch interessante bathochrome Nuancenverschiebung aus.

Als lineare Polyester, zu deren Einfärbung die erfindungsgemässen polymerlöslichen Pyrrolo[3,4-c]pyrrole besonders geeignet sind, seien insbesondere jene erwähnt, die durch Polykondensation von Terephthalsäure oder deren Estern mit Glykolen der Formel HO-(CH₂)ₙ-OH, worin n die Zahl 2-10 bedeutet, oder mit 1,4-Di-(hydroxymethyl)-cyclohexan oder durch Polykondensation von Glykolethern von Hydroxybenzoesäuren, beispielsweise p-(β-Hydroxyethoxy)-benzoesäure erhalten werden. Der Begriff lineare Polyester umfasst auch Kopolyester, die durch teilweisen Ersatz des Glykols durch ein anderes Diol erhalten werden. Von besonderem Interesse sind jedoch die Polyethylenterephthalate.

Die zu färbenden linearen Polyester werden zweckmässig in Form von Pulver, Schnitzeln oder Granulaten mit dem Farbstoff innig vermischt. Dies kann beispielsweise durch Panieren der Polyesterteilchen mit dem fein verteilten trockenen Farbstoffpulver oder durch Behandeln der Polyesterteilchen mit einer Lösung bzw. Dispersion des Farbstoffes in einem organischen Lösungsmittel und nachheriger Entfernung des Lösungsmittels geschehen.

Zur Nuanceeinstellung können Gemische der Pyrrolo[3,4-c]pyrrole der Formel I sowie Gemische einer oder mehrerer Verbindungen der Formel I mit Dispersionsfarbstoffen verwendet werden.

Schliesslich kann das Pyrrolo[3,4-c]pyrrol der Formel I auch direkt dem geschmolzenen Polyester oder auch vor oder während der Polykondensation des Polyäthylenterephthalates zugegeben werden.

Das Verhältnis von Farbstoff zu Polyester kann, je nach der gewünschten Farbstärke, innerhalb weiter Grenzen schwanken. Im allgemeinen empfiehlt sich die Verwendung von 0,01-3 Teilen Farbstoff auf 100 Teile Polyester.

Die so behandelten Polyesterteilchen werden nach bekannten Verfahren im Extruder geschmolzen und zu Gegenständen, insbesondere Folien oder Fasern, ausgepresst oder zu Platten gegossen.

Im Falle der Verwendung als Pigmente ist es vorteilhaft, die bei der Synthese anfallenden Produkte in eine feindisperse Form überzuführen. Dies kann auf verschiedene Weise geschehen, beispielsweise:
a) durch Mahlen oder Kneten, zweckmässig in Gegenwart von Mahlhilfsmitteln, wie anorganischen oder organischen Salzen mit oder ohne Zusatz organischer Lösungsmittel. Nach dem Mahlen werden die Hilfsmittel wie üblich entfernt, lösliche anorganische Salze z.B. mit Wasser und wasserunlösliche organische Lösungsmittel beispielsweise durch Wasserdampfdestillation,
b) durch Umfällen aus Schwefelsäure, Methansulfonsäure, Trichloressigsäure oder Polyphosphorsäure.
c) im Falle von Produkten, in denen D und/oder E Wasserstoff sind, durch Überführen des Rohpigments in ein Alkali- oder Aminsalz und Hydrolyse des letzteren. Dies geschieht beispielsweise durch Anrühren des Rohpigments mit einer Base, beispielsweise mit einem Alkalihydroxid, oder -alkoholat, Ammoniak oder einem Amin in einem polaren organischen Lösungsmittel wie Dimethylformamid, wobei das Pigment ganz oder teilweise in Lösung geht. Durch Hydrolyse, vorzugsweise durch Ansäuern der gegebenenfalls filtrierten Lösung wird das Pigment ausgefällt.

Es kann sich als zweckmässig erweisen, die nach a), b) oder c) behandelten Pigmente mit organischen Lösungsmitteln, vorzugsweise mit solchen, die über 100°C sieden, nachzubehandeln.

Als besonders geeignet erweisen sich durch Halogenatome, Alkyl- oder Nitrogruppen substituierte Benzole, wie Xylole, Chlorbenzol, o-Dichlorbenzol oder Nitrobenzol, sowie Pyridinbasen, wie Pyridin, Picolin oder Chinolin, ferner Ketone wie Cyclohexanon, Ether wie Ethylenglykolmonomethyl- oder monoethylether, Amide wie Dimethylformamid oder N-Methyl-pyrrolidon, sowie Dimethylsulfoxid, Sulfolan oder Wasser allein, gegebenenfalls unter Druck. Man kann die Nachbehandlung auch in Wasser in Gegenwart von organischen Lösungsmitteln und/oder mit Zusatz von oberflächenaktiven Substanzen, oder aliphatischen Aminen oder mit flüssigem Ammoniak durchführen.

Je nach Verwendungszweck erweist es sich als vorteilhaft, die Pigmente als solche (Toner) oder in Form von Präparaten zu verwenden.

Das hochmolekulare organische Material kann natürlicher oder künstlicher Herkunft sein. Es kann sich z.B. um Naturharze oder trocknende Oele, Kautschuk oder Casein oder um abgewandelte Naturstoffe, wie Chlorkautschuk, ölmodifizierte Alkydharze, Viscose, um Celluloseether oder Ester, wie Celluloseacetat, Cellulosepropionat, Celluloseacetobutyrat oder Nitrocellulose handeln, insbesondere aber um vollsynthetische organische Polymere (Duroplaste und Thermoplaste), wie sie durch Polymerisation, Polykondensation oder Polyaddition erhalten werden. Aus der Klasse der Polymerisationsharze seien in erster Linie genannt, Polyolefine, wie Polyethylen, Polypropylen, oder Polyisobutylen, ferner substituierte Polyolefine, wie Polymerisate aus Vinylchlorid, Vinylacetat, Styrol, Acrylnitril, Acrylsäure- und/oder Methacrylsäureestern oder Butadien, sowie Kopolymerisate der erwähnten Monomeren, insbesondere ABS oder EVA.

Aus der Reihe der Polyadditions- und Polykondensationsharze seien die Kondensationsprodukte von Formaldehyd mit Phenolen, die sogenannten Phenoplaste, und die Kondensationsprodukte von Formaldehyd mit Harnstoff, Thioharnstoff und Melamin, die sogenannten Aminoplaste, die als Lackharze verwendeten Polyester, und zwar sowohl gesättigte, wie z.B. Alkydharze, als auch ungesättigte, wie beispielsweise Maleinatharze, ferner die linearen Polyester und Polyamide oder Silikone, genannt.

Die erwähnten hochmolekularen Verbindungen können einzeln oder in Gemischen, als plastische Massen oder Schmelzen, die gegebenenfalls zu Fasern versponnen werden können, vorliegen.

Sie können auch in Form ihrer Monomeren oder im polymerisierten Zustand in gelöster Form als Filmbildner oder Bindemittel für Lacke oder Druckfarben vorliegen, wie z.B. Leinölfirnis, Nitrocellulose, Alkydharze, Melaminharze und Harnstoff-Formaldehydharce oder Acrylharze.

Die Pigmentierung der hochmolekularen, organischen Substanzen mit den Pigmenten der Formel (I) erfolgt beispielsweise derart, dass man ein solches Pigment gegebenenfalls in Form von Masterbatches diesen Substraten unter Verwendung von Walzwerken, Mischoder Mahlapparaten zumischt. Das pigmentierte Material wird hierauf nach an sich bekannten Verfahren wie Kalandrieren, Pressen, Strangpressen, Streichen, Giessen oder Spritzguss in die gewünschte endgültige Form gebracht. Oft ist es erwünscht, zur Herstellung von nicht starren Formlingen oder zur Verringerung ihrer Sprödigkeit den hochmolekularen Verbindungen vor der Verformung sogenannte Weichmacher einzuverleiben. Als solche können z.B. Ester der Phosphorsäure, Phthalsäure oder Sebacinsäure dienen. Die Weichmacher können im erfindungsgemässen Verfahren vor oder nach der Einverleibung des Pigmentfarbstoffes in die Polymeren eingearbeitet werden. Es ist ferner möglich, zwecks Erzielung verschiedener Farbtöne den hochmolekularen, organischen Stoffen neben den Pyrrolo[3,4-c]pyrrolen der Formel (I) noch Füllstoffe bzw. andere farbgebende Bestandteile wie Weiss-, Bunt- oder Schwarzpigmente in beliebigen Mengen zuzufügen.

Zum Pigmentieren von Lacken und Druckfarben werden die hochmolekularen organischen Materialien und die Pyrrolo[3,4-c]pyrrole der Formel (I) gegebenenfalls zusammen mit Zusatzstoffen wie Füllmitteln, anderen Pigmenten, Siccativen oder Weichmachern, in einem gemeinsamen organischen Lösungsmittel oder Lösungsmittelgemisch fein dispergiert bzw. gelöst. Man kann dabei so verfahren, dass man die einzelnen Komponenten für sich oder auch mehrere gemeinsam dispergiert bzw. löst, und erst hierauf alle Komponenten zusammenbringt.

Die erhaltenen Färbungen, beispielsweise in Kunststoffen, Fasern, Lacken oder Drucken, zeichnen sich durch einen gelben bis roten Farbton, eine sehr grosse Farbstärke, hohe Sättigung, gute Dispergierbarkeit, gute Überlackier-, Migrations-, Hitze-, Licht- und Wetterechtheit sowie durch einen guten Glanz und gutes IR-Remissionsverhalten aus. Ganz charakteristisch ist, wie bereits erwähnt, die überraschend hohe Festkörperfluoreszenz.

Die Pyrrolo[3,4-c]pyrrole der Formel (I) können auch als Toner für Elektro- und Magnetographie verwendet werden. Ferner können sie als Farbmittel für Drucktinten, insbesondere für Tintenstrahl- sowie Sicherheitsdruck, eingesetzt werden.

Wenn die Pyrrolo[3,4-c]pyrrole der Formel (I) in den angewandten Polymeren gelöst vorliegen, zeichnen sie sich ebenfalls durch reinen Farbton, grosse Farbstärke, gute Echtheiten, insbesondere Licht- und Sublimierechtheit, und ausserdem durch hohe Fluoreszenz aus. Sie eignen sich zur Verwendung in Sonnenenergie-Kollektoren und zur Induktion von Laser-Strahlen. Ausserdem sind sie als organische Photoleiter für Kopiermaschinen und Laserdrucker sowie als Aktivkomponenten von Elektrolumineszenz-Elementen gut geeignet.

Die nachfolgenden Beispiele dienen zur Erläuterung der Erfindung.

Beispiel 1: Zu einer Lösung von 7,03 ml (64 mMol) TiCl₄ in 30 ml 1,2-Dichlorbenzol, unter Argon, wird eine Lösung von 14,48 ml (64 mMol) Bis(trimethylsilyl)carbodiimid in 30 ml 1,2-Dichlorbenzol gegeben. Zu der entstandenen roten Lösung wird nach 30 Minuten eine Suspension von 1,5 g (3,2 mMol) N,N'-Dimethyl-1,4-diketo-3,6-diphenylpyrrolo[3,4-c]-pyrrol in 30 ml 1,2-Dichlorbenzol gegeben. Die dunkelbraune Suspension wird bei 80°C 8 Tage gerührt und danach in 400 g Eiswasser gegossen, dann abfiltriert und der Niederschlag wird mit Wasser und anschliessend mit Aceton gewaschen und an der Luft getrocknet. Das rote Pulver wird aus 700 ml Benzonitril bei 155°C umkristallisiert. Das Kristallisat wird abfiltriert und mit Benzonitril gewaschen. Man erhält 1,105 g (67 %) bordeaux-rotes fluoreszierendes Pulver von N,N'-Dimethyl-1,4-dicyanimino-3,6-diphenylpyrrolo[3,4-c]-pyrrol vom Schmelzpunkt 328,6°C.

UV (Benzonitril): λₘₐₓ 530 nm.

| Analyse: | C | H | N |
|---|---|---|---|
| Ber. | 79,05 % | 4,68 % | 16,27 % |
| Gef. | 76,53 % | 4,77 % | 16,17 % |

Beispiele 2-14: Setzt man bei gleicher Arbeitsweise, wie in Beispiel 1 beschrieben den entsprechenden 1,4-Diketo-pyrrolo[3,4-c]-pyrrol mit der gewünschten Menge Bis(trimethylsilyl)carbodiimid nach folgendem Schema um, so erhält man die in der nachstehenden Tabelle 1 aufgezeichneten Produkte.

Ansatzmengen der Ausgangsprodukte, λₘₐₓ und Smp. werden für die jeweiligen Beispiele in Tabelle 2 aufgeführt.

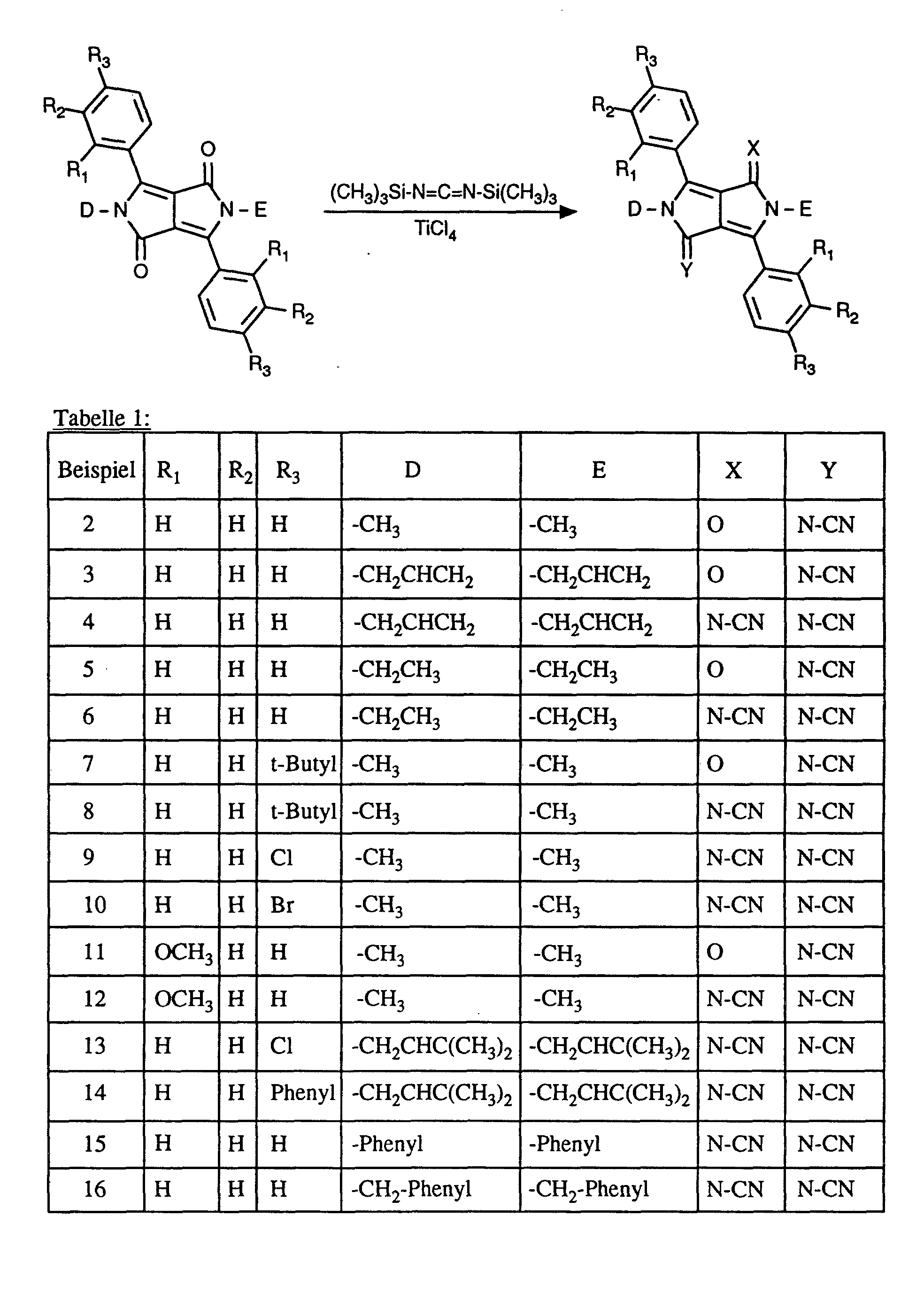

| | Ansatzmengen | | UV λmax/CH₂Cl₂ | Smp. |
|---|---|---|---|---|
| | 1,4-Diketopyrrolopyrrol | Carbodiimid | | |
| 2 | 12,64 mMol | 126,4 mMol | 491 nm | 205,6°C |
| 3 | 1,36 mMol | 13,6 mMol | 488 nm | 169,3°C |
| 4 | 1,36 mMol | 13,6 mMol | 513 nm | 227,6°C |
| 5 | 16,06 mMol | 160,5 mMol | 488 nm | 259,4°C |
| 6 | 16,06 mMol | 160,5 mMol | 514 nm | 326,8°C |
| 7 | 1,16 mMol | 11,6 mMol | 495 nm | 305,8°C |
| 8 | 1,16 mMol | 11,6 mMol | 520 nm | Zs 387°C |
| 9 | 3,9 mMol | 78 mMol | 524 nm | Zs 361°C |
| 10 | 2,2 mMol | 42,2 mMol | 524 nm | Zs 368°C |
| 11 | 1,22 mMol | 13,3 mMol | 487 nm | 179,4°C |
| 12 | 1,22 mMol | 13,3 mMol | 520 nm | Zs 283°C |
| 13 | 0,41 mMol | 4,1 mMol | 521 nm | 262,3°C |
| 14 | 2,27 mMol | 9,08 mMol | 521 nm | 260,9°C |
| 15 | 1,12 mMol | 11,2 mMol | 444 nm | 170°C |
| 16 | 2,5 mMol | 25 mMol | 430 nm | 272°C |

## Patentansprüche

1. Pyrrolo[3,4-c]pyrrole der Formel 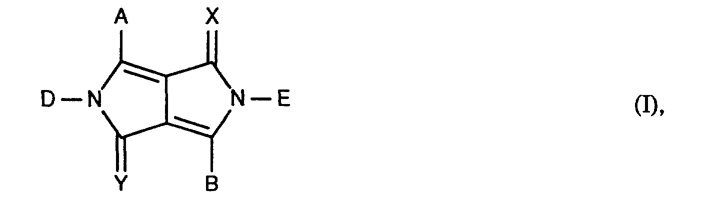 worin A und B unabhängig voneinander für eine Gruppe der Formel 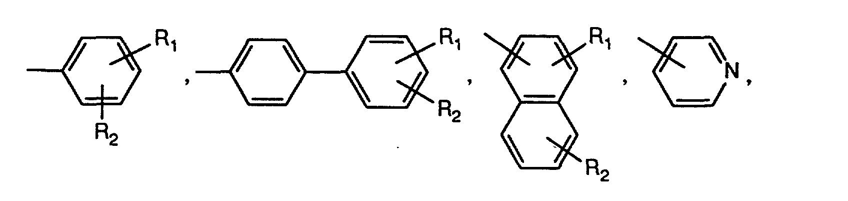 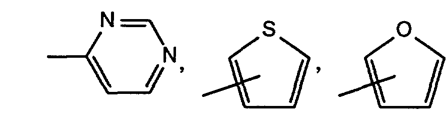 oder 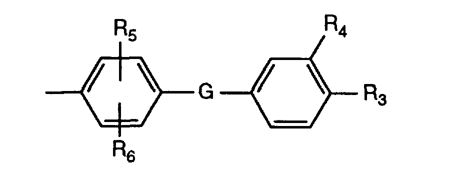 stehen, worin
R₁ und R₂ unabhängig voneinander Wasserstoff, Halogen, C₁-C₁₈-Alkyl, C₁-C₁₈-Alkoxy, C₁-C₁₈-Alkylmercapto, C₁-C₁₈-Alkylamino, -CN, -NO₂, -Phenyl, Trifluormethyl, C₅-C₆-Cycloalkyl, -C=N-(C₁-C₁₈-Alkyl), 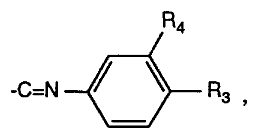 Imidazolyl, Pyrazolyl, Triazolyl, Piperazinyl, Pyrrolyl, Oxazolyl, Benzoxazolyl, Benzthiazolyl, Benzimidazolyl, Morpholinyl, Piperidinyl oder Pyrrolidinyl,
R₃ und R₄ unabhängig voneinander Wasserstoff, Halogen, C₁-C₆-Alkyl, C₁-C₁₈-Alkoxy oder -CN,
R₅ und R₆ unabhängig voneinander Wasserstoff, Halogen oder C₁-C₆-Alkyl, und
G -CH₂-, -CH(CH₃)-, -C(CH₃)₂-, -CH=N-, -N=N-, -O-, -S-, -SO-, -SO₂- oder -NR₇-, worin R₇ Wasserstoff oder C₁-C₆-Alkyl ist, bedeuten,
D und E unabhängig voneinander Wasserstoff, C₁-C₁₈-Alkyl, C₂-C₄-Alkenyl, C₇-C₁₀-Aralkyl, unsubstituiertes oder durch Chlor, Brom, C₁-C₆-Alkyl, C₁-C₄-Alkoxy, Trifluormethyl oder Nitro substituiertes Phenyl, -COO-C₁-C₅-Alkyl oder eine Gruppe -COOR₈,
worin R₈ Benzyl, Piperidyl oder eine Gruppe 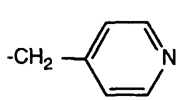 oder 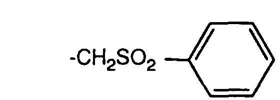 ist, bedeuten, und
X und Y für N-CN oder O stehen, wobei mindestens einer von X oder Y N-CN sein muss.

2. Pyrrolo[3,4-c]pyrrole gemäss Anspruch 1, **dadurch gekennzeichnet, dass** in der Formel I A und B unabhängig voneinander eine Gruppe der Formel 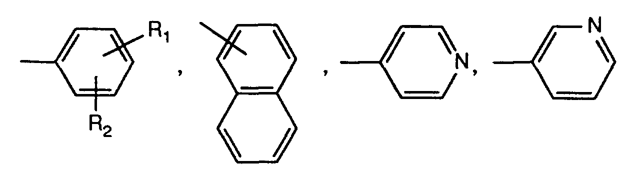 oder 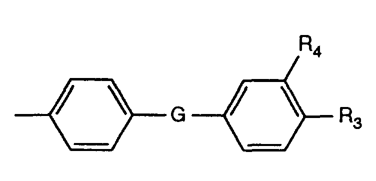 sind, worin R₁ und R₂ unabhängig voneinander Wasserstoff, Chlor, Brom, C₁-C₄-Alkyl, C₁-C₆-Alkoxy, C₁-C₆-Alkylamino, CN oder Phenyl,
R₃ und R₄ Wasserstoff, und
G -O-, -NR₇-, -N=N- oder -SO₂-, worin R₇ Wasserstoff, Methyl oder Ethyl ist, bedeuten.

3. Pyrrolo[3,4-c]pyrrole gemäss Anspruch 1, **dadurch gekennzeichnet, dass** in der Formel I A und B gleich sind.

4. Pyrrolo[3,4-c]pyrrole gemäss Anspruch 1, **dadurch gekennzeichnet, dass** in der Formel I A und B eine Gruppe der Formel 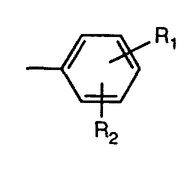 sind, worin R₁ und R₂ unabhängig voneinander Wasserstoff, Methyl, tert.-Butyl, Chlor, Brom, CN oder Phenyl bedeuten.

5. Pyrrolo[3,4-c]pyrrole gemäss Anspruch 1, **dadurch gekennzeichnet, dass** in der Formel I D und E gleich sind und C₁-C₄-Alkyl, unsubstituiertes oder durch Chlor substituiertes Phenyl, Allyl oder Benzyl oder eine Gruppe -COO-C(CH₃)₃, 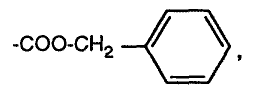 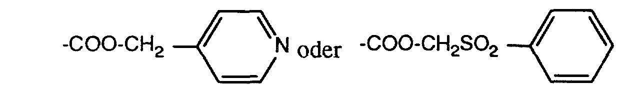 bedeuten.

6. Pyrrolo[3,4-c]pyrrole gemäss Anspruch 5, **dadurch gekennzeichnet, dass** in der Formel I D und E C₁-C₄-Alkyl oder eine Gruppe -COO-C(CH₃)₃ bedeuten.

7. Pyrrolo[3,4-c]pyrrole gemäss Anspruch 1, **dadurch gekennzeichnet, dass** in der Formel I X und Y N-CN bedeuten.

8. Verfahren zur Herstellung von Pyrrolo[3,4-c]pyrrolen gemäss Anspruch 1 der Formel I, worin A, B, X und Y die in Anspruch 1 angegebene Bedeutung haben und D und E unabhängig voneinander Wasserstoff, C₁-C₁₈-Alkyl, C₂-C₄-Alkenyl, C₇-C₁₀-Aralkyl, unsubstituiertes oder durch Chlor, Brom, C₁-C₆-Alkyl, C₁-C₄-Alkoxy, Trifluormethyl oder Nitro substituiertes Phenyl bedeuten,
durch Umsetzung eines Pyrrolo[3,4-c]pyrrols der Formel 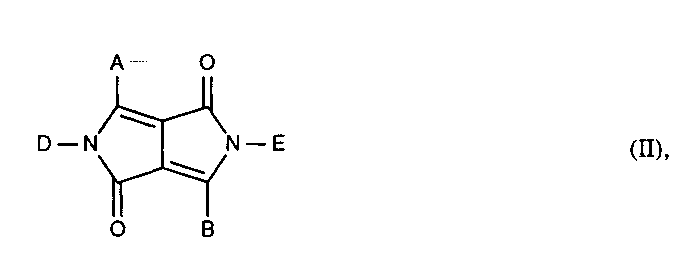 worin A und B die in Anspruch 1 und D und E die eben in diesem Anspruch angegebene Bedeutung haben, im gewünschten Molverhältnis mit einer Verbindung der Formel
(R₉)₃SiN=C=NSi(R₉)₃ (III)
oder
NH₂CN (IV)
wobei R₉ C₁-C₆-Alkyl bedeutet,
in Gegenwart einer Lewis-Säure als Katalysator in einem aprotischen organischen Lösungsmittel, bei Temperaturen zwischen 10 und 150°C.

9. Verfahren zur Herstellung von Pyrrolo[3,4-c]pyrrolen gemäss Anspruch 1 der Formel I, worin D und/oder E -COO-C₁-C₅-Alkyl oder eine Gruppe -COOR₈, worin R₈ Benzyl, Piperidyl oder eine Gruppe 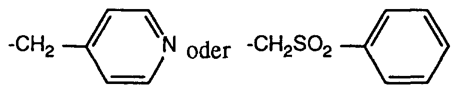 ist, bedeuten, durch Umsetzung eines Pyrrolo[3,4-c]pyrrols der Formel I, worin D und E Wasserstoff bedeuten, im gewünschten Molverhältnis mit einem Dicarbonat der Formel
E-O-E (V)
oder mit einem 1:1 Gemisch von Dicarbonat der Formel V und Dicarbonat der Formel
D - O - D (VI),
worin E und D unabhängig voneinander -COO-C₁-C₅-Alkyl oder eine Gruppe -COOR₈, worin R₈ die oben angegebene Bedeutung hat, bedeuten.

10. Verwendung von Verbindungen (I), gemäss Anspruch 1, als Fluoreszenzfarbstoffe in einem hochmolekularen, organischen Material.

## Claims

1. A pyrrolo[3,4-c]pyrrole of formula 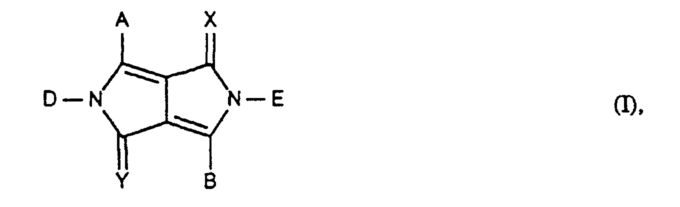 in which A and B are each independently of the other a group of formula 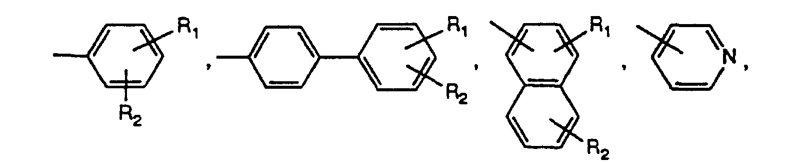 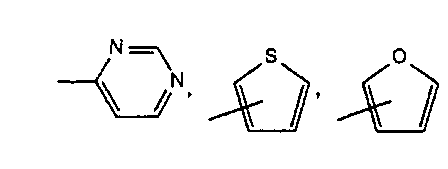 or 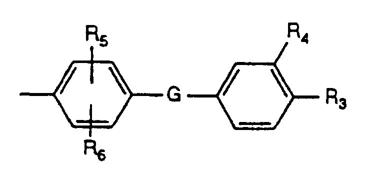 in which
R₁ and R₂ are each independently of the other hydrogen, halogen, C₁-C₁₈alkyl, C₁-C₁₈alkoxy, C₁-C₁₈alkylmercapto, C₁-C₁₈alkylamino, -CN, -NO₂, phenyl, trifluoromethyl, C₅-C₆cycloalkyl, -C=N- (C₁-C₁₈alkyl), 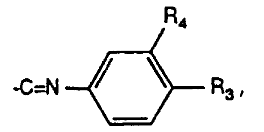 imidazolyl, pyrazolyl, triazolyl, piperazinyl, pyrrolyl, oxazolyl, benzoxazolyl, benzothiazolyl, benzimidazolyl, morpholinyl, piperidinyl or pyrrolidinyl,
R₃ and R₄ are each independently of the other hydrogen, halogen, C₁-C₆alkyl, C₁-C₁₈alkoxy or -CN,
R₅ and R₆ are each independently of the other hydrogen, halogen or C₁-C₆alkyl, and G is -CH₂-, -CH(CH₃)-, -(CH₃)₂-, -CH=N-, -N=N-, -O-, -S-, -SO-, -SO₂- or -NR₇-, in which R₇ is hydrogen or C₁-C₆alkyl,
D and E are each independently of the other hydrogen, C₁-C₁₈alkyl, C₂-C₄alkenyl, C₇-C₁₀aralkyl, unsubstituted phenyl or phenyl which is substituted by chloro, bromo, C₁-C₆alkyl, C₁-C₄alkoxy, trifluoromethyl or nitro; -COO-C₁-C₅alkyl or a group -COOR₈,
in which R₈ is benzyl, piperidyl or a group 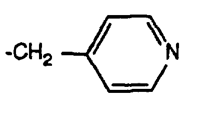 or 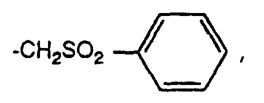 and
X and Y are N-CN or O, with the proviso that at least one of X or Y must be N-CN.

2. A pyrrolo[3,4-c]pyrrole according to claim 1, wherein A and B in formula I are each independently of the other a group of formula 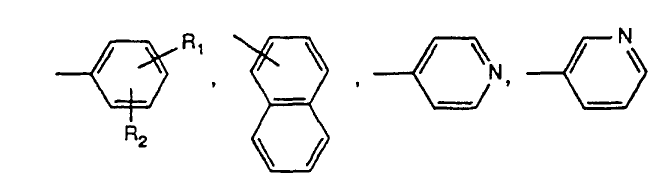 or 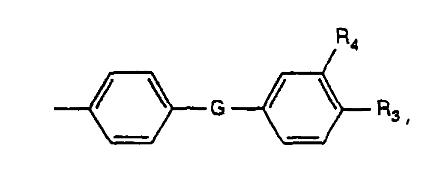 in which R₁ and R₂ are each independently of the other hydrogen, chloro, bromo, C₁-C₄alkyl, C₁-C₆alkoxy, C₁-C₆alkylamino, CN or phenyl,
R₃ and R₄ are hydrogen, and
G is -O-, -NR₇-, -N=N- or -SO₂-, in which R₇ is hydrogen, methyl or ethyl.

3. A pyrrolo[3,4-c]pyrrole according to claim 1, wherein A and B in formula I are identical.

4. A pyrrolo[3,4-c]pyrrole according to claim 1, wherein A and B in formula I are a group of formula 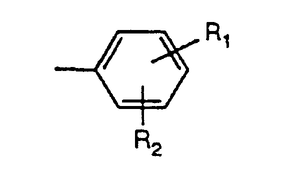 in which R₁ and R₂ are each independently of the other hydrogen, methyl, tert-butyl, chloro, bromo, CN or phenyl.

5. A pyrrolo[3,4-c]pyrrole according to claim 1, wherein D and E in formula I are identical and are C₁-C₄alkyl, unsubstituted or chloro-substituted phenyl, allyl or benzyl or a group - COO-C(CH₃)₃, 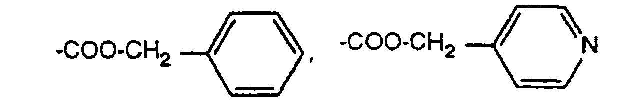 or

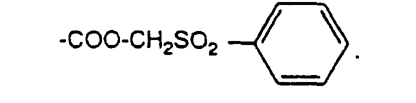

6. A pyrrolo[3,4-c]pyrrole according to claim 5, wherein D and E in formula 1 are C₁-C₄alkyl or a group -COO-C(CH₃)₃.

7. A pyrrolo[3,4-c]pyrrole according to claim 1, wherein X and Y in formula I are N-CN.

8. A process for the preparation of a pyrrolo [3,4-c]pyrrole of formula I according to claim 1, in which A, B, X and Y are as defined in claim 1 and D and E are each independently of the other hydrogen, C₁-C₁₈alkyl, C₂-C₄alkenyl, C₇-C₁₀aralkyl, unsubstituted phenyl or phenyl which is substituted by chloro, bromo, C₁-C₆alkyl, C₁-C₄alkoxy, trifluoromethyl or nitro, by reacting a pyrrolo[3,4-c]pyrrole of formula 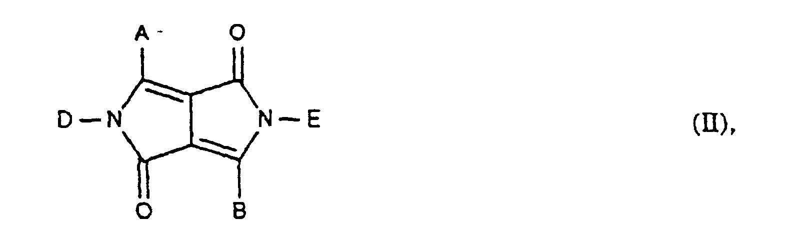 in which A and B are as defined in claim 1 and D and E have the meaning just given in this claim, in the desired molar ratio with a compound of formula
(R₉)₃SiN=C=NSi(R₉)₃ (III)
or
NH₂CN (IV)
where R₉ is C₁-C₆alkyl,
in the presence of a Lewis acid as catalyst in an aprotic organic solvent, at temperatures between 10 and 150°C.

9. A process for the preparation of a pyrrolo [3,4-c]pyrrole of formula I according to claim 1, in which D and/or E is -COO-C₁-C₅alkyl or a group -COOR₈, in which R₈ is benzyl, piperidyl or a group 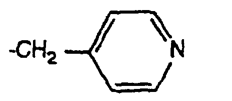 or 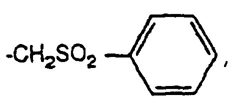 by reacting a pyrrolo[3,4-c]pyrrole of formula I in which D and E are hydrogen, in the desired molar ratio, with a dicarbonate of formula
E - O - E (V)
or with a 1:1 mixture of dicarbonate of formula V and dicarbonate of formula
D - O - D (VI),
in which E and D are each independently of the other -COO-C₁-C₅alkyl or a group -COOR₈, in which R₈ has the above meaning.

10. Use of compounds (I) according to claim 1 as fluorescent dyes in a high molecular weight organic material.

## Revendications

1. Pyrrolo[3,4-c]pyrroles de formule 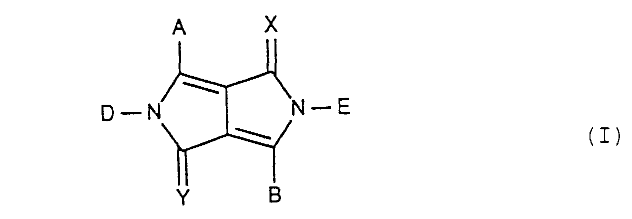 où A et B représentent, indépendamment l'un de l'autre, un groupe de formule 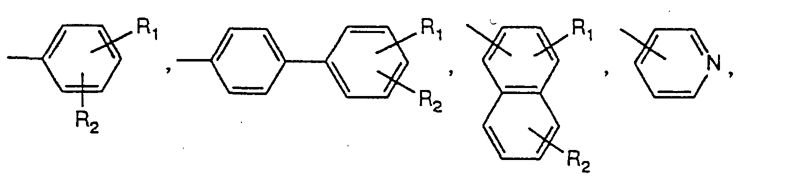 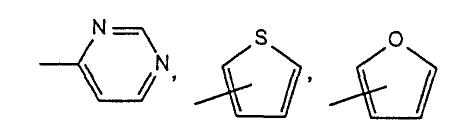 ou 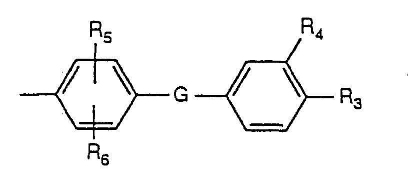 où R₁ et R₂ représentent, indépendamment l'un de l'autre, des atomes d'hydrogène, des atomes d'halogènes, des groupes alkyle en C₁-C₁₈, alkoxy en C₁-C₁₈, (alkyl en C₁-C₁₈)mercapto, (alkyl en C₁-C₁₈)-amino, -CN, -NO₂, phényle, trifluorométhyle, cycloalkyle en C₅-C₆, -C=N-(alkyle en C₁-C₁₈) 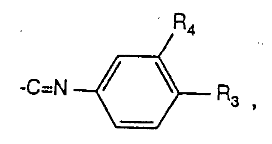 imidazolyle, pyrazolyle, triazolyle, pipérazinyle, pyrrolyle, oxazolyle, benzoxazolyle, benzthiazolyle, benzimidazolyle, morpholinyle, pipéridinyle ou pyrrolidinyle,
R₃ et R₄ représentent, indépendamment l'un de l'autre, des atomes d'hydrogène, des atomes d'halogènes, des groupes alkyle en C₁-C₆, alkoxy en C₁-C₁₈ ou -CN,
R₅ et R₆ représentent, indépendamment l'un de l'autre, un atome d'hydrogène, un atome d'halogène ou un groupe alkyle en C₁-C₆, et
G représente des groupes -CH₂-, -CH(CH₃)-, -(CH₃)₂-, -CH=N-, -N=N-, -O-, -S-, -SO-, -SO₂- ou -NR₇-, où
R₇ représente un atome d'hydrogène ou un groupe alkyle en C₁-C₆,
D et E représentent, indépendamment l'un de l'autre, des atomes d'hydrogène, des groupes alkyle en C₁-C₁₈, alcényle en C₂-C₄, aralkyle en C₇-C₁₀, phényle non substitué ou substitué par des substituants chloro, bromo, alkyle en C₁-C₆, alkoxy en C₁-C₄, trifluorométhyle ou nitro, un groupe -COO- alkyle en C₁-C₅ ou un groupe -COOR₈, où R₈ représente des groupes benzyle, pipéridyle ou un groupe 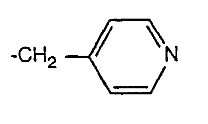 ou 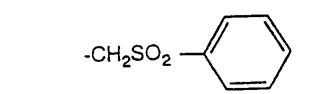 et
X et Y représentent des groupes N-CN ou O, au moins un des X ou Y devant être un groupe N-CN.

2. Pyrrolo[3,4-c]pyrroles selon la revendication 1, **caractérisés en ce que** à la formule I, A et B représentent, indépendamment l'un de l'autre, un groupe de formule 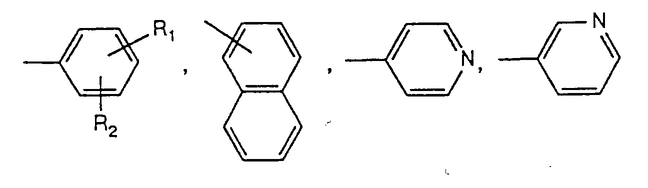 ou 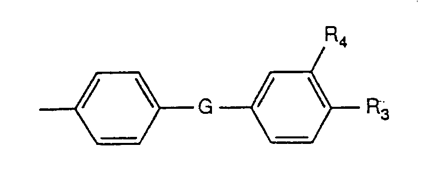 où R₁ et R₂ représentent, indépendamment l'un de l'autre, des atomes d'hydrogène, de chlore, de brome, des goupes alkyle en C₁-C₄, alkoxy en C₁-C₆, (alkyl en C₁-C₆)amino, -CN ou phényle,
R₃ et R₄ représentent, indépendamment l'un de l'autre, des atomes d'hydrogène, et
G représente des groupes -O-, -NR₇-, -N=N-, ou -SO₂-, où R₇ représente un atome d'hydrogène, des groupes méthyle ou éthyle,

3. Pyrrolo[3,4-c]pyrroles selon la revendication 1, **caractérisés en ce que** à la formule I A et B sont identiques.

4. Pyrrolo[3,4-c]pyrroles selon la revendication 1, **caractérisés en ce que** à la formule I A et B représentent un groupe de formule 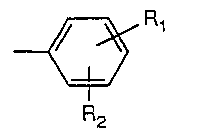 où R₁ et R₂ représentent, indépendamment l'un de l'autre, un atome d'hydrogène, des groupes méthyle, tert.-butyle, chloro, bromo, CN ou phényle.

5. Pyrrolo[3,4-c]pyrroles selon la revendication 1, **caractérisés en ce que** à la formule I D et E sont identiques et représentent des groupes alkyle en C₁-C₄, benzyle, allyle ou phényle non substitué ou substitué par un substituant chloro, ou un groupe -COO-C(CH₃)₃,

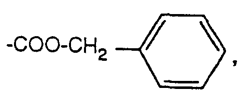

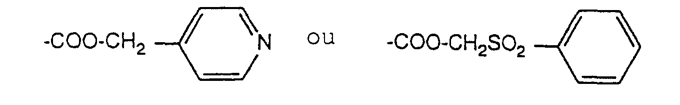

6. Pyrrolo[3,4-c]pyrroles selon la revendication 5, **caractérisés en ce que** à la formule I, D et E représentent un groupe alkyle en C₁-C₄ ou un groupe -COO-C(CH₃)₃.

7. Pyrrolo[3,4-c]pyrroles selon la revendication 1, **caractérisés en ce que** à la formule I X et Y représentent un groupe N-CN.

8. Procédé pour la préparation de pyrrolo[3,4-c]pyrroles de formule I, selon la revendication 1, où A, B, X et Y possèdent la signification donnée à la revendication 1 et D et E représentent, indépendamment l'un de l'autre, des atomes d'hydrogène, des groupes alkyle en C₁-C₁₈, alcényle en C₂-C₄, aralkyle en C₇-C₁₀, phényle non substitué ou substitué par des substituants chloro, bromo, alkyle en C₁-C₆, alkoxy en C₁-C₄, trifluorométhyle ou nitro,
par réaction d'un pyrrolo[3,4-c]pyrrole de formule 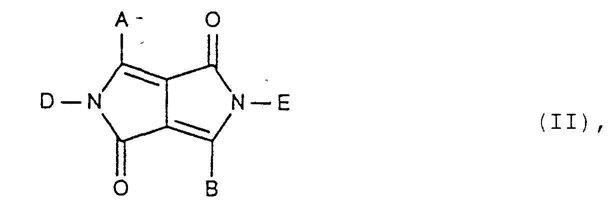 où A et B possèdent la signification donnée à la revendication 1 et D et E possèdent également la signification donnée à cette revendication, dans le rapport molaire souhaité sur un composé de formule
(R₉)₃SiN=C=NSi(R₉)₃ (III)
ou
NH₂CN (IV)
où R₉ représente un groupe alkyle en C₁-C₆,
en présence d'un acide de Lewis en tant que catalyseur dans un solvant organique aprotique, à une température comprise entre 10 et 150°C.

9. Procédé pour la préparation de pyrrolo[3,4-c]pyrroles de formule I selon la revendication 1, où D et/ou E représentent un groupe - COO-alkyle en C₁-C₅ ou un groupe -COOR₈, où R₈ représente un groupe benzyle, pipéridyle ou un groupe 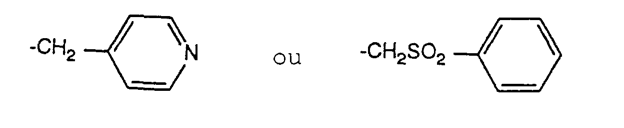 par réaction d'un pyrrolo[3,4-c]pyrrole de formule I, où D et E représdentent un atome d'hydrogène, dans le rapport molaire souhaité, sur un dicarbonate de formule
E-O-E (V)
ou sur un mélange 1:1 de dicarbonate de formule V et de dicarbonate de formule
D-O-D (VI)
où E et D représentent, indépendamment l'un de l'autre, un groupe -COO-alkyle en C₁-C₅ ou un groupe -COOR₈, où R₈ possède la signification donnée ci-dessus.

10. Utilisation de composés de formule (I) selon la revendication 1, en tant que colorants fluorescents dans une matière organique de haut poids moléculaire.
